# EUROPEAN PATENT APPLICATION

(11) **EP 4 596 681 A1**
(43) Date of publication of application: **06.08.2025**
(21) Application number: 23872595.6
(22) Date of filing: 29.09.2023
(51) Int. Cl.: C12N 5/079, C12N 5/10, C12N 15/12, C12Q 1/02

(54) **HUMAN ASTROCYTE CELL MASS, CELL MASS CULTURE, METHOD FOR PRODUCING HUMAN ASTROCYTE CELL MASS, AND METHOD FOR EVALUATING TEST SUBSTANCE**

(30) Priority: 30.09.2022 JP 2022157559; 30.06.2023 JP 2023108776
(71) Applicant: FUJIFILM Corporation, Tokyo 106-8620 (JP)
(72) Inventor: KOBAYASHI, Hayato, Ashigarakami-gun, Kanagawa 258-8577 (JP); ENDOH, Setsu, Ashigarakami-gun, Kanagawa 258-8577 (JP); NABETANI, Akira, Ashigarakami-gun, Kanagawa 258-8577 (JP); KATO, Hiroshi, Ashigarakami-gun, Kanagawa 258-8577 (JP)
(74) Representative: Hoffmann Eitle
(86) International application number: PCT/JP2023/035597
(87) International publication number: WO 2024/071375

(57) **Abstract**

An object of the present invention is to provide a human astrocyte cell population that is differentiated from astrocyte progenitor cells derived from human iPS cells, a manufacturing method for the human astrocyte cell population; and an evaluation method for a test substance using the human astrocyte cell population. According to the present invention, there is provided a human astrocyte cell population that is differentiated from astrocyte progenitor cells derived from human iPS cells, the human astrocyte cell population including at least 90% of human astrocytes, in which in the human astrocytes, a) CDKN2Ais positive, b) at least one gene marker selected from the group consisting of IGFBP5, NNMT, HLA-DRB1, and HLA-DRB5 is positive, and c) an expression level of C3, which is standardized with GAPDH of a reference gene, is 0.05 copies/copies or less.

## Description

### Technical Field

The present invention relates to a human astrocyte cell population, a cell population culture product, and a manufacturing method for a human astrocyte cell population. The present invention further relates to an evaluation method for a test substance using the manufactured human astrocyte cell population. Furthermore, the present invention relates to a manufacturing method for a co-culture product containing the manufactured human astrocyte cell population, human-derived nerve cells, and human-derived microglia, and the co-culture product.

### Background Art

Astrocytes, which are a type of glial cells that constitutes the brain, play various roles in maintaining homeostasis of the central nervous system, such as supplying nutrients to neurons and forming or removing synapses, and are attracting attention from the viewpoint of elucidating the mechanism of diseases and drug discovery.

Cells of the central nervous system composed of nerve cells and glial cells are difficult to acquire from the human brain, and in many studies on the central nervous system, cells of rats or mice have been used. However, in recent years, because of the invention of iPS cells, it has become possible to relatively easily acquire human nerve cells and glial cells, and these cells are very useful for research on human neurodegenerative diseases (Non-Patent Documents 1 to 3).

In the research on neurodegenerative diseases, it is very important to consider the influence of cellular senescence associated with aging, which is the greatest risk factor for diseases (Non-Patent Document 4). It is also known that the cellular senescence of astrocytes is involved in neurodegenerative diseases (Non-Patent Documents 5 to 7), and research has been conducted to reproduce senescent astrocytes (Non-Patent Document 8). However, since the senescence is reset by reprogramming in iPS cells (Non-Patent Documents 9 and 10), it is considered that it is difficult to reproduce the aging.

In Non-Patent Document 11, research has been conducted on iPS cell-derived astrocytes, in which senescence is reproduced by producing astrocytes from patient-derived iPS cells with mutations in disease-related genes. However, in the method of Non-Patent Document 11, since the astrocytes are completely differentiated and then cultured for a long period of time, it is unclear whether or not senescent astrocytes can be obtained.

In addition, although it is common to culture astrocytes in a culture medium containing serum, it has been recently known that exposure of serum to astrocytes causes irreversible activation (Non-Patent Document 12). It is considered that astrocytes in the brain are in a state of being unexposed to serum because of the presence of the blood-brain barrier (Non-Patent Document 13), and it is important to culture astrocytes under conditions without serum to study the function of astrocytes (Non-Patent Document 14).

Patent Document 1 and Patent Document 2 describe a method of culturing astrocytes under conditions in which serum is not contained. However, it is described in the method of Patent Document 1 that the differentiation into neurons and oligodendrocytes other than astrocytes occurs, and furthermore it is unclear whether the differentiated astrocytes are senescent astrocytes. In addition, it is described in the method of Patent Document 2 that A1 astrocytes having neurotoxic properties can be obtained, but it is unclear whether or not senescent astrocytes having no neurotoxic properties can be obtained.

Patent Document 3 describes a method of preparing astrocyte-like cells from human cells. However, since astrocytes are differentiated in a short period of time in the method of Patent Document 3, it is unclear whether or not senescent astrocytes can be obtained.

### Prior Art Documents

### Patent Documents

Patent Document 1: WO2021/045217A
Patent Document 2: WO2019/235576A
Patent Document 3: WO2017/057523A

### Non-Patent Documents

Non-Patent Document 1: Okano et al., Molecular Brain, vol. 7, no. 22, pp. 1 to 12, 2014
Non-Patent Document 2: Valadez-Barba et al., Regenerative Therapy, vol. 15, pp. 332 to 339, 2020
Non-Patent Document 3: Zeng et al., STEM CELLS TRANSLATIONAL MEDICINE, vol. 3, pp. 1418 to 1428, 2014
Non-Patent Document 4: Kritsilis et al., International Journal of Molecular Sciences, vol. 19, no. 10, 2937, pp. 1 to 37, 2018
Non-Patent Document 5: Turnquist et al., Cell Death and Differentiation, vol. 23, pp. 1515 to 1528, 2016
Non-Patent Document 6: Han et al., Frontiers in Aging Neuroscience, vol. 12, no. 148, 2020
Non-Patent Document 7: Vazquez-Villasenor et al., Neuropathology and Applied Neurobiology, vol. 46, pp. 171 to 185, 2020
Non-Patent Document 8: Simmnacher et al., Experimental Neurology, vol. 334, no. 113466, pp. 1 to 13, 2020
Non-Patent Document 9: Lapasset et al., GENES & DEVELOPMENT, vol. 25, no. 21, pp. 2248 to 2253, 2011
Non-Patent Document 10: Miller et al., Cell Stem Cell, Vol. 13, No. 6, pp. 691 to 705, 2013
Non-Patent Document 11: Birger et al., EBioMedicine, vol. 50, pp. 274 to 289, 2019
Non-Patent Document 12: Foo et al., Neuron, vol. 71, no. 5, pp. 799 to 811, 2011
Non-Patent Document 13: Stokum et al., Neurochemical Research, vol. 40, pp. 317 to 328, 2015
Non-Patent Document 14: Jia et al., Journal of Neuroscience Methods, vol. 307, pp. 240 to 247, 2018

### SUMMARY OF THE INVENTION

### Object to be solved by the invention

Human senescent astrocytes have a great value from the viewpoint of drug discovery research, and a method for easily obtaining senescent human astrocytes is demanded. As described above, certain results have been obtained regarding the differentiation induction into astrocytes, but a method for reliably inducing from astrocyte progenitor cells derived from human iPS cells into senescent human astrocytes has not been found.

Therefore, an object to be achieved by the present invention is to provide a human astrocyte cell population including a senescent human astrocyte induced from astrocyte progenitor cells derived from human iPS cells. Another object to be achieved by the present invention is to provide a cell population culture product containing the human astrocyte cell population, and a manufacturing method for the human astrocyte cell population. Another object to be achieved by the present invention is to provide an evaluation method for a test substance using the human astrocyte cell population. Another object to be achieved by the present invention is to provide a manufacturing method for a co-culture product containing the human astrocyte cell population, human-derived nerve cells, and human-derived microglia, and the co-culture product.

### Means for solving the object

As a result of intensive studies to achieve the above objects, the present inventors have found that the astrocyte progenitor cells derived from human iPS cells can be induced to senescent human astrocytes by aging the astrocyte progenitor cells derived from human iPS cells under a proliferation condition. The present invention has been completed based on these findings.

That is, according to an aspect of the present invention, the following invention is provided.
<1> A human astrocyte cell population that is differentiated from astrocyte progenitor cells derived from human iPS cells, the human astrocyte cell population comprising:
   at least 90% of human astrocytes,
   in which in the human astrocytes,
      a) CDKN2A is positive,
      b) at least one marker selected from the group consisting of IGFBP5, NNMT, HLA-DRB1, and HLA-DRB5 is positive, and
      c) an expression level of C3, which is standardized with GAPDH of a reference gene, is 0.05 copies/copies or less.
<2> The human astrocyte cell population according to <1>, in which in the human astrocytes, an expression level of CDKN2A, which is standardized with GAPDH of the reference gene, is 0.004 copies/copies or more.
<3> The human astrocyte cell population according to <1> or <2>, in which in the human astrocytes, an expression level of IGFBP5, which is standardized with GAPDH of the reference gene, is 0.1 copies/copies or more.
<4> The human astrocyte cell population according to <1> or <2>, in which in the human astrocytes, an expression level of NNMT, which is standardized with GAPDH of the reference gene, is 0.005 copies/copies or more.
<5> The human astrocyte cell population according to <1> or <2>, in which in the human astrocytes, an expression level of HLA-DRB5, which is standardized with GAPDH of the reference gene, is 0.1 copies/copies or more.
<6> The human astrocyte cell population according to any one of <1> to <5>, in which at least one gene marker selected from the group consisting of γH2AX and SA-β-GAL is positive.
<7> The cell population according to any one of <1> to <6>, in which the astrocyte progenitor cells derived from human iPS cells are astrocyte progenitor cells produced from human iPS cells derived from a healthy person.
<8> A cell population culture product comprising:
   the human astrocyte cell population according to any one of <1> to <7>; and
   a culture medium that does not substantially contain serum.
<9> The cell population culture product according to <8>, further comprising:
   at least one factor selected from the group consisting of bone morphogenetic protein 4 (BMP4) and ciliary neurotrophic factor (CNTF).
<10> A manufacturing method for the astrocyte cell population according to any one of <1> to <7>, further comprising:
   proliferating astrocyte progenitor cells derived from human iPS cells; and
   inducing the differentiation of the proliferated astrocyte progenitor cells derived from human iPS cells.
<11> An evaluation method for a test substance, comprising:
   bringing the human astrocyte cell population according to any one of <1> to <7> into contact with a test substance.
<12> A manufacturing method for a co-culture product, comprising:
   a step of adding the human astrocyte cell population according to any one of <1> to <7>, human-derived nerve cells, and human-derived microglia to a culture container; and
   a step of co-culturing the human astrocyte cell population, the nerve cells, and the microglia in the culture container.
<13> The manufacturing method according to <12>, in which the nerve cells and the microglia are obtained by inducing differentiation from human-derived pluripotent stem cells.
<14> The manufacturing method according to <12> or <13>, in which human-derived pluripotent stem cells are human iPS cells.
<15> The manufacturing method according to any one of <12> to <14>, in which the co-culture product is a two-dimensional culture product or a three-dimensional culture product.
<16> A co-culture product which is obtained by the manufacturing method according to <12> to <15>; comprising:
   the human astrocyte cell population according to <1> to <7>;
   human-derived nerve cells; and
   human-derived microglia.
   Effect of the Invention

According to the present invention, it is possible to manufacture a senescent human astrocyte cell population from astrocyte progenitor cells derived from human iPS cells. The human astrocyte cell population according to the embodiment of the present invention and the evaluation method for a test substance according to the embodiment of the present invention are useful in drug discovery research and the like.

### BRIEF DESCRIPTION OF THE DRAWINGS

Fig. 1 shows an image obtained by performing immunofluorescence staining on a senescent human astrocyte with an anti-GFAP antibody.
Fig. 2 shows results of quantifying a GFAP positive rate of senescent human astrocytes using a flow cytometer.
Fig. 3 shows results of quantifying expression levels of senescence-associated markers for senescent human astrocytes and non-senescent human astrocyte using digital PCR.
Fig. 4 shows results of quantifying C3 expression level in senescent human astrocytes and non-senescent human astrocytes using digital PCR.
Fig. 5 shows results of immunofluorescence staining with an anti-γH2AX antibody for senescent human astrocytes and non-senescent human astrocytes.
Fig. 6 shows results of staining SA-β-GAL of senescent human astrocytes and non-senescent human astrocytes.

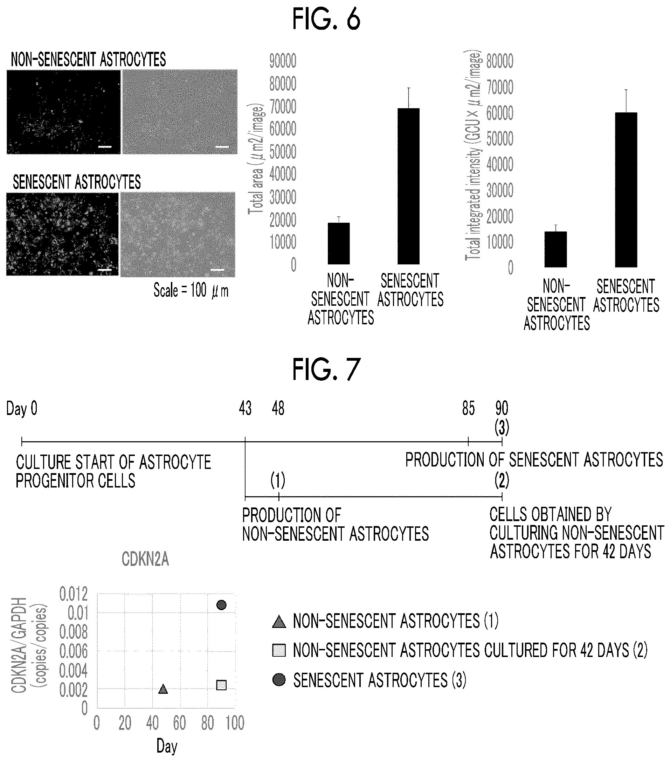

Fig. 7 shows results of quantifying CDKN2A expression level in senescent human astrocytes, senescent human astrocytes cultured for a long period of time, and non-senescent human astrocytes using digital PCR. The triangle indicates ((1)) cells obtained by culturing an astrocyte progenitor cell in a progenitor cell culture medium for 43 days and then replacing the culture medium with a differentiation-inducing culture medium to induce differentiation into an astrocyte. The square indicates ((2)) cells obtained by culturing non-senescent astrocytes for 42 days. The circle indicates ((3)) cells obtained by culturing an astrocyte progenitor cell in a progenitor cell culture medium for 85 days and then replacing the culture medium with a differentiation-inducing culture medium to induce differentiation into an astrocyte.
Fig. 8 shows an image obtained by performing immunofluorescence staining on a two-dimensional co-culture product produced using senescent human astrocytes, nerve cells, and microglia.
Fig. 9 shows an image obtained by performing immunofluorescence staining on a three-dimensional co-culture product produced using senescent human astrocytes, nerve cells, and microglia.

### EMBODIMENTS FOR CARRYING OUT THE INVENTION

Hereinafter, embodiments of the present invention will be specifically described.
CDKN2A indicates cyclin-dependent kinase inhibitor 2A.
IGFBP5 indicates insulin-like growth factor binding protein 5.
NNMT indicates nicotinamide N-methyltransferase.
HLA-DRB1 indicates human leukocyte antigen-DRB1.
HLA-DRB5 indicates human leukocyte antigen-DRB5.
C3 indicates complement molecule C3.
GAPDH indicates glyceraldehyde 3-phosphate dehydrogenase.
BMP4 indicates bone morphogenetic protein 4.
CNTF indicates ciliary neurotrophic factor.
GFAP indicates glial fibrillary acidic protein.
γH2AX indicates phosphorylated histone H2AX.
SA-β-GAL indicates senescence-associated beta-galactosidase.

The present invention relates to a human astrocyte cell population that is differentiated from astrocyte progenitor cells derived from human iPS cells.

The human astrocyte cell population according to the embodiment of the present invention includes at least 90% of human astrocytes, in which in the human astrocytes,
a) CDKN2A is positive,
b) at least one marker selected from the group consisting of IGFBP5, NNMT, HLA-DRB 1, and HLA-DRB5 is positive, and
c) an expression level of C3, which is standardized with GAPDH of a reference gene, is 0.05 copies/copies or less.

### <Human iPS cell>

The "human iPS cell" is an induced pluripotent stem cell produced from a human cell.

In the present specification, the term "iPS cell" means a cell that is produced by reprogramming a somatic cell by introducing reprogramming factors and has pluripotency (multiple differentiation potency) and proliferation ability. The iPS cells exhibit properties similar to those of embryonic stem cells (ES cells). The somatic cells used for producing iPS cells are not particularly limited and may be differentiated somatic cells or undifferentiated stem cells. In addition, the origin thereof is not particularly limited; however, it is preferable to use a somatic cell of mammals (for example, primates such as a human and a chimpanzee, rodents such as a mouse and a rat), it is more preferable to use a human somatic cell. The iPS cell can be prepared by a known method or the like. In addition, it is naturally expected that an iPS cell production method to be developed in the future will be applied.

The most basic producing method for an iPS cell is a method in which four transcription factors, Oct3/4, Sox2, Klf4, and c-Myc are introduced into a cell using a virus (Takahashi K., Yamanaka S., Cell 126 (4), 663-676, 2006, Takahashi, K., et al., Cell 131 (5), 861-72, 2007). It has been reported that human iPS cells have been established by introducing four factors, Oct4, Sox2, Lin28, and Nanog (Yu J, et al., Science 318 (5858), 1917-1920, 2007). It has also been reported that iPS cells have been established by introducing three factors excluding c-Myc (Nakagawa M, et al., Nat. Biotechnol. 26 (1), 101-106, 2008), two factors of Oct3/4 and Klf4 (Kim J. B, et al., Nature 454 (7204), 646-650, 2008), or Oct3/4 alone (Kim J B, et al., Cell 136 (3), 411-419, 2009). In addition, a method for introducing a protein, which is an expression product of a gene, into cells (Zhou H, Wu S, Joo J Y, et al., Cell Stem Cell 4, 381-384, 2009, Kim D, Kim C H, Moon J I, et al., Cell Stem Cell 4, 472-476, 2009) has also been reported.

As the producing method of iPS cells, in addition to the method of manufacturing an iPS cell by direct initialization by gene expression, it is also possible to induce an iPS cell from a somatic cell by addition of a compound or the like (Hou P et al., Science 341(6146), 651-654, 2013).

In addition, it is also possible to obtain an established induced pluripotent stem cell, and they can be provided, for example, from National University Corporation Kyoto University, iPS Academia Japan, Inc., or Institute of Physical and Chemical Research BioResource Research Center.

A cell in which the transformation to an iPS cell, that is, the initialization has occurred can be selected using the expression of pluripotent stem cell markers (undifferentiated markers) such as Nanog, Oct/4, Fgf-4, Esg-1, and Cript as an indicator, and the selected cell can be collected as an iPS cell.

### <Astrocyte progenitor cells derived from human iPS cells>

The "Astrocyte progenitor cells derived from human iPS cells" are astrocyte progenitor cells produced from human iPS cells.

In the present specification, the term "astrocyte progenitor cell" means a cell having an ability of differentiating into an astrocyte. The presence of the astrocyte progenitor cell can be specified by a marker that is significantly recognized to be expressed in the astrocyte progenitor cell. Examples of the marker of the astrocyte progenitor cell include NFIA, NFIB, SOX9, HEY1, HEY2, FABP7, ZBTB20, and the like.

Examples of the method for obtaining the "astrocyte progenitor cell" used in the present invention include obtaining the astrocyte progenitor cell by separating from the cerebral cortex, spinal cord, or the like surgically acquired from a patient, inducing from a cell that can differentiate into human astrocytes, and inducing from human pluripotent stem cells. All of these astrocyte progenitor cells can be used as the "astrocyte progenitor cell" in the method according to the embodiment of the present invention.

As the "astrocyte progenitor cell" used in the present invention, it is preferable to use a human astrocyte progenitor cell induced from a human pluripotent stem cell.

Examples of the human pluripotent stem cell include human induced pluripotent stem cells (human iPS cells), human embryonic stem cells (human ES cells), and human mesenchymal stem cells. The human pluripotent stem cell is not particularly limited, but human iPS cells are preferably used.

That is, according to the present invention, there is provided a human astrocyte cell population that is differentiated from an astrocyte progenitor cell, the human astrocyte cell population including at least 90% of human astrocytes, in which in the human astrocytes,
a) CDKN2A is positive,
b) at least one marker selected from the group consisting of IGFBP5, NNMT, HLA-DRB 1, and HLA-DRB5 is positive, and
c) an expression level of C3, which is standardized with GAPDH of a reference gene, is 0.05 copies/copies or less.

Examples of the method for obtaining the "astrocyte progenitor cells derived from human iPS cells" include inducing from human iPS cells produced from somatic cells collected from healthy humans (healthy person) who do not have a mutation of a disease related gene causing a nervous system disease or who do not have a nervous system disease, or from somatic cells collected from patients who have a nervous system disease, and inducing from established human iPS cells. The nervous system disease is not particularly limited, and examples thereof include Alzheimer's disease, amyotrophic lateral sclerosis (ALS), Parkinson's disease, autism, Alexander disease, Rett syndrome, and the like.

All of these astrocyte progenitor cells derived from human iPS cells can be used as "astrocyte progenitor cells derived from human iPS cells" which are the starting cells in the present invention.

As the "astrocyte progenitor cells derived from human iPS cells" used in the present invention, astrocyte progenitor cells induced from human iPS cells derived from a healthy person, astrocyte progenitor cells induced from established human iPS cells, or astrocyte progenitor cells induced from human iPS cells having no gene mutation derived from a disease is preferably used, and astrocyte progenitor cells produced from human iPS cells derived from a healthy person is more preferably used.

The astrocyte progenitor cells derived from human iPS cells can be used in the present invention even in a state of being isolated or in a state of being mixed with other cells. In addition, it can be assumed that the differentiation induction from human iPS cells into astrocyte progenitor cells and the differentiation induction from astrocyte progenitor cells into human astrocytes are continuously performed.

### <Human astrocytes>

"Human astrocytes" are astrocytes of human.

In the present specification, the "astrocyte" is a type of glial cell present in the central nervous system, and is considered to contribute to the regulation of nerve transmission by having a function of structurally supporting a neuron or regulating a neurotransmitter, or energy and extracellular ions. In addition, it is considered that the astrocytes supply a substance that promotes myelin formation to oligodendrocytes, which are also a type of glial cells, and are cells that play an important role together with neurons and oligodendrocytes in nervous tissues. The astrocytes can be identified by a marker that is specifically expressed in astrocytes, and as the marker, for example, GFAP, S100 calcium binding protein B (S100B), potassium inwardly rectifying channel subfamily J member 10 (KCNJ10), Aquaporin-4 (AQP4), and solute carrier family 1 member 3 (SLC1A3, also referred to as GLAST or EAAT1), and the like are known. For example, by analyzing using an immunohistological method, it is possible to discriminate cells expressing at least one of the mRNA, the sugar chain, or the protein as astrocytes.

### <Human astrocyte cell population>

The "human astrocyte cell population" is a population of cells including human astrocytes.

In the present specification, the "cell population" is a population containing at least one type of cell, and may contain any two or more types of cells. In addition, the cell population may be a cell population in a state of being dispersed (floating) in a medium such as a culture medium, in a state of being adhered to a bottom surface of a culture container, in a state of a cell aggregation (spherical cell population) in which a plurality of cells are aggregated, or in a state of being layered, and is not particularly limited.

The "human astrocyte cell population" according to the embodiment of the present invention preferably contains at least 90% of human astrocytes, more preferably contains at least 95% of human astrocytes, still more preferably contains at least 99% of human astrocytes, and particularly preferably contains 100% of human astrocytes.

The proportion of human astrocytes in the human astrocyte cell population can be determined, for example, by quantifying the GFAP positive rate with a flow cytometer using an anti-GFAP antibody as an antibody that specifically recognizes human astrocytes.

Examples of the cells other than human astrocytes contained in the human astrocyte cell population according to the embodiment of the present invention include astrocyte progenitor cells, neural stem cells, oligodendrocyte progenitor cells, nerve cells, oligodendrocytes, and the like, but the cells are not particularly limited.

### <Senescent human astrocytes>

The "senescent human astrocytes (human senescent astrocytes)" are human astrocytes in which the expression of senescence-associated markers, which are characteristic of cellular senescence, is positive.

The senescence-associated marker is not particularly limited, and examples thereof include γH2AX, which is known as a DNA damage response (DDR) marker; CDKN2A (p16INK4a and p14ARF), p21, and p53, which are known as a tumor suppressor or a cell cycle regulator; senescence-associated β-galactosidase (SA-β-GAL) known as a lysosome-related protein; and inflammatory cytokines IL-6 and IL-8 and a vascular endothelial growth factor (VEGF) which are known as a senescence-associated secretory phenotype (SASP) marker, and the like.

In the present specification, in the "senescent human astrocytes", for example, it is preferable that at least one selected from the group consisting of CDKN2A, γH2AX, SA-β-GAL, and SASP markers, which are a senescence-associated marker, is positive, more preferable that at least one selected from the group consisting of the CDKN2A, the γH2AX, and the SA-β-GAL is positive, and still more preferable that the CDKN2A is positive.

In the present specification, the "marker" means a substance that is present in a cell and can identify or discriminate the type, properties, or the like of the cell based on the presence or the abundance of the substance. Specific examples of the marker include mRNA, a protein and a sugar chain encoded by the mRNA, and fragments thereof.

In the present specification, the "the marker is positive" indicates that the expression level of the marker in the human astrocytes contained in the human astrocyte cell population according to the embodiment of the present invention, which is the final product, is high as compared with the expression level of the marker in the starting cell.

In a case where the marker is a gene, the expression level of the marker means the expression level (expression amount) of the gene, and can be analyzed by the production amount of the transcript corresponding to the gene, the production amount, activity, or the like of the translation product thereof. The measurement of the expression level can be performed by measuring an mRNA which is a transcript of a gene or a protein which is a translation product of a gene; however, it is preferably carried out by measuring an mRNA or a cDNA which is a reverse transcript thereof. The detection or measurement of the expression of the translation product (protein) can be performed by immunocytochemistry for detecting the protein in the cell using an antibody.

Table 1 shows the NCBI accession number of each gene. Based on the following NCBI accession number, the sequence information of each gene can be acquired, and the expression level of the gene can be measured.

**[Table 1]**

| Gene name | NCBI accession Number |
|---|---|
| CDKN2A | NM_000077, NM_058197, NM_058195, NM_ 001195132, NM_001363763 |
| IGFBP5 | NM_000599 |
| NNMT | NM_006169, NM_001372045, NM_001372046, NM_001372047 |
| HLA-DRB1 | NM_002124, NM_001243965,NM_001359193, NM_001359194 |
| HLA-DRB5 | NM_002125 |
| C3 | NM_000064 |

The measuring method for the expression level of the gene expressed by the human astrocytes contained in the human astrocyte cell population according to the embodiment of the present invention is not particularly limited, and for example, the measurement can be performed by quantitative RT-PCR. The RT-PCR is a method of synthesizing cDNA using the measurement target mRNA as a template and amplifying it by PCR using this cDNA as a template. Examples of the quantitative RT-PCR include a method (real-time PCR) of performing PCR using a primer to which a quencher fluorescent dye and a reporter fluorescent dye are bound, quantifying the amount of an amplification product for each cycle, and measuring the amount of template DNA in a sample from the number of cycles in which the detected fluorescence intensity rapidly increases, a method (digital PCR) of dispersing a limit-diluted sample DNA in a microcompartment, performing PCR amplification, and directly counting the number of microcompartments containing a target gene to absolutely quantify the concentration of the target gene in the sample, and the like. Examples of the method of dispersing the sample DNA in the microcompartments by the digital PCR include a method of producing droplets, a method of dispersing the sample DNA on a chip, and the like, but the method is not particularly limited. The quantitative RT-PCR technique is well known in the technical field of the present invention and can also be carried out using a commercially available kit. According to the quantitative RT-PCR, the expression amount or the number of copies of a gene can be measured as a relative value with respect to the expression amount or the number of copies of a control reference gene (for example, a GAPDH gene). The mRNA of a gene can also be measured by subjecting an amplification product obtained by amplifying the mRNA by the ordinary RT-PCR or the like to gel electrophoresis, staining the gel, and then measuring the band intensity. Alternatively, a DNA chip can be used to detect or quantify the mRNA or cDNA of a gene. The expression level of a gene expressed by the human astrocytes can also be measured using a next-generation sequencer. A measurement target cell can be obtained by the partial extraction of cells in the culture step.

As the numerical value indicating the expression level, for example, in a case where the expression level is measured by real-time PCR, a cycle threshold (Ct) value can be used. The Ct value is the number of cycles at which a PCR amplification product reaches a certain amount. In a case where the number of cycles of amplification is plotted on the horizontal axis and the amount of a PCR product is plotted on the vertical axis to create an amplification curve, and a threshold is set for the value of the PCR product, the number of cycles at the point where the threshold and the amplification curve intersect is the Ct value. In a case of measuring the expression amount using a fluorescently labeled probe, fluorescence intensity can also be used.

In a case where the expression level is measured by digital PCR, the number of copies (copies/µL) can be used. The number of microcompartments containing the target gene (positive) and the number of microcompartments not containing the target gene (negative) are counted, and the number of copies is calculated from the proportion of negative compartments. The number of target genes contained in the positive can be calculated using a Poisson distribution, and the number of copies can be corrected.

### <CDKN2A>

"CDKN2A" is also referred to as cyclin dependent kinase inhibitor 2A (CDK2A), and is a cancer suppressor gene that encodes p16INK4a (p16) and p14ARF (p14).

It is preferable that CDKN2A is positive in the human astrocytes contained in the human astrocyte cell population according to the embodiment of the present invention.

In the present invention, in a case where CDKN2A is positive, the expression level of CDKN2A in the human astrocytes, which is standardized by GAPDH of the reference gene, is preferably 0.002 copies/copies or more, more preferably 0.003 copies/copies or more, still more preferably 0.004 copies/copies or more, even still more preferably 0.005 copies/copies or more, and particularly preferably 0.006 copies/copies or more.

In the human astrocytes, the upper limit of the expression level of CDKN2A standardized with GAPDH of the reference gene is not particularly limited, and the higher the expression level is, the more it is assumed that the human astrocytes contained in the human astrocyte cell population according to the embodiment of the present invention are senescent.

In the human astrocytes contained in the human astrocyte cell population according to the embodiment of the present invention, in addition to the fact that CDKN2A is positive, at least one marker selected from the group consisting of IGFBP5, NNMT, HLA-DRB1, and HLA-DRB5 may be positive, and at least one marker selected from the group consisting of the IGFBP5, the NNMT, the HLA-DRB 1, and the HLA-DRB5 is preferably positive, at least one marker selected from the group consisting of the IGFBP5, the NNMT, and the HLA-DRB5 is more preferably positive, at least one marker selected from the group consisting of the IGFBP5 and the NNMT is still more preferably positive, and the IGFBP5 is even still more preferably positive.

### <IGFBP5>

"IGFBP5" is also referred to as insulin-like growth factor binding protein 5, and is one of the proteins that are present in blood and tissues by binding to insulin-like growth factors (IGFs). It is considered that IGF binds to an insulin-like growth factor binding protein (IGFBP) to regulate the affinity to a receptor, the distribution (local release), and the metabolism (stabilization and decomposition). IGFBPs bind to IGF-I and IGF-II that circulate in body fluids to regulate the activity (promotion and suppression), distribution (local release), and metabolism (stabilization and decomposition). In addition to IGF-BPs having a high affinity for IGFs, an IGF-BP related Protein (IGFBPrP) that have structural and functional similarities but have a low affinity have been identified. IGFBP5 is mainly produced in vascular smooth muscle cells, and is localized in bone tissue to promote the action of IGF-I on smooth muscle cells, fibroblasts, and osteoblasts. In addition, it is known to form a trimer with an acidic-labile subunit (ALC) in the same manner as IGFBP3. In addition, it is known as a senescence-associated secretory phenotype (SASP) regulator.

In the human astrocytes contained in the human astrocyte cell population according to the embodiment of the present invention, IGFBP5 may be positive and IGFBP5 is preferably positive.

In the present invention, in a case where IGFBP5 is positive, the expression level of IGFBP5 in the human astrocytes, which is standardized by GAPDH of the reference gene, is preferably 0.08 copies/copies or more, more preferably 0.09 copies/copies or more, still more preferably 0.1 copies/copies or more, even still more preferably 0.15 copies/copies or more, and particularly preferably 0.2 copies/copies or more.

In the human astrocytes, the upper limit of the expression level of IGFBP5 standardized with GAPDH of the reference gene is not particularly limited, and the higher the expression level is, the more it is assumed that the human astrocytes contained in the human astrocyte cell population according to the embodiment of the present invention are senescent.

### <NNMT>

"NNMT" is also referred to as nicotinamide N-methyltransferase, and is an enzyme that catalyzes the methylation of nicotinamide and similar compounds using S-adenosylmethionine, which is a methyl donor, to produce S-adenosyl-L-homocysteine and 1-methylnicotinamide. In addition, it is known to be an essential contributing factor for various metabolic processes including aging, cell stress response, and body weight gain regulation, and epigenetic processes.

In the human astrocytes contained in the human astrocyte cell population according to the embodiment of the present invention, NNMT may be positive and NNMT is preferably positive.

In the present invention, in a case where NNMT is positive, the expression level of NNMT in the human astrocytes, which is standardized by GAPDH of the reference gene, is preferably 0.003 copies/copies or more, more preferably 0.004 copies/copies or more, still more preferably 0.005 copies/copies or more, even still more preferably 0.01 copies/copies or more, and particularly preferably 0.015 copies/copies or more.

In the human astrocytes, the upper limit of the expression level of NNMT standardized with GAPDH of the reference gene is not particularly limited, and the higher the expression level is, the more it is assumed that the human astrocytes contained in the human astrocyte cell population according to the embodiment of the present invention are senescent.

### <HLA-DRB1 and HLA-DRB5>

"HLA-DRB1" is one of haplotypes of human leukocyte antigen (HLA) that is known to function as a histocompatibility antigen and is also referred to as human leukocyte antigen-DRB1 (HLA-DRB1) and major histocompatibility complex class II DRβ1. HLA-DRB1 is a protein-coding gene located in an HLA class II region on the short arm of the sixth chromosome. The HLA class II molecule is a heterodimer consisting of an alpha (DRA) chain and a beta (DRB) chain, both of which are fixed to the membrane and display a peptide derived from extracellular proteins, thereby playing a central role in the immune system.

As diseases associated with HLA-DRB1, multiple sclerosis, sarcoidosis 1, and the like are known and pathways associated therewith include D28 signaling in helper T cells, and the like. In addition, HLA-DRB5 is known as an important paralog of the gene of HLA-DRB1.

"HLA-DRBS" is also referred to as human leukocyte antigen-DRB5 or major histocompatibility complex class II DRβ5, and is one of the haplotypes of HLA, similar to HLA-DRB1.

As diseases associated with HLA-DRB5, pityriasis rosea, which is inflammatory keratosis (scaly rash) of the skin commonly seen in young adults, and multiple epiphyseal dysplasia due to an abnormality in collagen 9, and the like are known, and pathways associated therewith include CD28 signaling in helper T cells, and the like. In addition, HLA-DRB1 is known as an important paralog of the gene of HLA-DRB5.

In the human astrocytes contained in the human astrocyte cell population according to the embodiment of the present invention, at least one marker selected from the group consisting of HLA-DRB1 and HLA-DRB5 may be positive, at least one marker selected from the group consisting of the HLA-DRB1 and the HLA-DRB5 is preferably positive, and the HLA-DRB5 is more preferably positive.

In the present invention, in a case where HLA-DRB5 is positive, the expression level of HLA-DRB5 in the human astrocytes, which is standardized by GAPDH of the reference gene, is preferably 0.08 copies/copies or more, more preferably 0.09 copies/copies or more, still more preferably 0.1 copies/copies or more, even still more preferably 0.15 copies/copies or more, particularly preferably 0.2 copies/copies or more, and more particularly preferably 0.25 copies/copies or more.

In the human astrocytes, the upper limit of the expression level of HLA-DRB5 standardized with GAPDH of the reference gene is not particularly limited, and the higher the expression level is, the more it is assumed that the human astrocytes contained in the human astrocyte cell population according to the embodiment of the present invention are senescent.

In the human astrocytes contained in the human astrocyte cell population according to the embodiment of the present invention, in addition to the fact that CDKN2A is positive, C3 may be negative and the expression level of C3, which is standardized with GAPDH of the reference gene, may be 0.05 copies/copies or less.

### <C3>

"C3" is also referred to as a complement molecule C3, and is one of the complements present in the serum of a mammal. The C3 is also known as a specific marker of human A1 astrocytes, which exhibit neurotoxic properties. Examples of the specific marker of human A1 astrocytes include GBP2, SERPING1, C3, and the like, but the marker is not limited thereto.

In the human astrocytes contained in the human astrocyte cell population according to the embodiment of the present invention, C3 is preferably negative.

In the present invention, in a case where C3 is negative, the expression level of C3 in the human astrocytes, which is standardized by GAPDH of the reference gene, is 0.05 copies/copies or less, more preferably 0.02 copies/copies or less, still more preferably 0.01 copies/copies or less, even still more preferably 0.001 copies/copies or less, particularly preferably 0.0001 copies/copies or less, and most preferably equal to or less than the detection limit.

In the human astrocytes, the lower limit of the expression level of C3 standardized with GAPDH of the reference gene is not particularly limited, and it is assumed that the higher the expression level is, the human astrocytes are cells having different properties from the human astrocytes contained in the human astrocyte cell population according to the embodiment of the present invention.

In the human astrocytes contained in the human astrocyte cell population according to the embodiment of the present invention, at least one marker selected from the group consisting of γH2AX and SA-β-GAL may be positive, and at least one marker selected from the group consisting of the γH2AX and the SA-β-GAL is preferably positive.

### <γH2AX>

"γH2AX" is also referred to as phosphorylated histone H2AX and is known as one of the markers for DNA damage. In a case where double-strand break occurs due to DNA damage, H2AX, which is a type of histone proteins, is rapidly and widely phosphorylated. γH2AX is known not only as an indicator for evaluating the genotoxicity and carcinogenicity of a chemical substance, active oxygen, ultraviolet rays, radiation, or the like but also as an indicator for evaluating cellular senescence in recent years.

### <SA-β-GAL>

"SA-β-GAL" is one of enzymes also referred to as senescence-associated beta-galactosidase. In a case where β-galactosidase is stained with X-gal as a substrate under weakly acidic conditions (pH 6), senescent cells are stained blue, whereas proliferating cells are not stained. Therefore, the SA-β-GAL is widely used as a marker that can easily detect senescent cells that have undergone cellular senescence.

As another aspect, the present invention relates to a cell population culture product containing a human astrocyte cell population that is differentiated from astrocyte progenitor cells derived from human iPS cells.

### <Cell population culture product>

The "cell population culture product" is a culture product that contains a cell population.

**In** the present specification, the "cell population culture product" may contain a cell population containing at least one type of cells, and any component such as a culture medium.

### <Culture of human astrocytes>

The culture of human astrocytes in the present invention may be performed by selecting a culture medium, a temperature, and other conditions according to the origin and the state of the human astrocytes to be used. Any component such as a factor suitable for the purpose of culture may be contained in the culture medium as long as the culture of human astrocytes in the present invention is not disturbed. A medium can be selected from the known media and commercially available media. For example, suitable components (serum, protein, amino acid, sugar, vitamin, fatty acid, antibiotics, and the like) are added to a general medium such as minimum essential medium (MEM), Dulbecco's modified Eagle medium (DMEM), DMEM/F12, or a medium obtained by modifying these media, for using in cell culture.

As the culture medium used in the present invention, it is preferable to use a culture medium that does not substantially contain serum (serum-free medium) from the purpose of obtaining an environment closer to in vivo to study the function of astrocytes, in which astrocytes in the brain are in a state of not being exposed to serum due to the presence of the blood-brain barrier.

In the present invention, the "serum-free medium" means a culture medium containing no unadjusted or unpurified serum. In the present specification, even a culture medium in which a purified blood-derived component or an animal tissue-derived component (for example, a growth factor) is mixed, is included in a serum-free medium as long as it contains no unadjusted or unpurified serum.

The cell population culture product in the present invention preferably contains a human astrocyte cell population containing human senescent astrocytes, and a culture medium that does not substantially contain serum.

The culture medium used in the present invention may contain any component such as a factor required for differentiation induction from human astrocyte progenitor cells into human astrocytes.

The cell population culture product in the present invention preferably further contains at least one factor selected from the group consisting of bone morphogenetic protein 4 (BMP4) and ciliary neurotrophic factor (CNTF) for the purpose of efficiently differentiating human astrocyte progenitor cells into human astrocytes.

### <Manufacturing method for human astrocyte cell population>

In another aspect, there is provided a manufacturing method for a cell population containing senescent human astrocytes.

According to the present invention, there is provided a manufacturing method for a human astrocyte cell population, including proliferation of astrocyte progenitor cells derived from human iPS cells and differentiation of the proliferated astrocyte progenitor cells derived from human iPS cells.

As the culture conditions in the manufacturing method according to the embodiment of the present invention, general cell culture conditions may be selected. Conditions of 37°C and 5% CO₂, and the like are exemplified. During the culture, it is preferable to change the medium at appropriate intervals (preferably once a day to 7 days and more preferably once every 2 days to 3 days). In a case where the cell population according to the embodiment of the present invention is produced using human astrocyte progenitor cells derived from iPS cells as a material, human senescent astrocytes can be produced by culturing the cells under a proliferation condition for 50 days or more and then differentiated into human astrocytes. The period of time for culturing under the proliferation conditions is preferably 50 days or more, more preferably 70 days or more, and still more preferably 80 days or more.

In addition, from the viewpoint of applying stress that induces senescence, it is preferable that the human astrocyte progenitor cells are subjected to two-dimensional culture.

For culturing the cells, cell culture containers such as plates, dishes, cell culture inserts, cell culture flasks, and cell culture bags can be used. As the cell culture bag, a cell culture bag having gas permeability is suitable. In a case where a large amount of cells is required, a large culture tank may be used. The culture can be performed in either of an open system or a closed system.

### <Human astrocyte cell population and evaluation method for test substance using the human astrocyte cell population>

According to the present invention, a cell population containing senescent human astrocytes are provided.

The human astrocyte cell population according to the embodiment of the present invention can also be used for screening a pharmaceutical candidate compound acting on human senescent astrocytes or for safety evaluation of a pharmaceutical candidate compound.

The present invention provides an evaluation method for a test substance, which includes bringing a test substance into contact with the cell population containing the human astrocytes according to the embodiment of the present invention. For example, by bringing the test substance into contact with the astrocyte progenitor cells in the step of culturing the astrocyte progenitor cells for a long period of time to induce senescence, it is possible to evaluate the antisenescence action. In addition, for example, by bringing a test substance into contact with a cell population containing the human astrocytes according to the embodiment of the present invention, it is possible to evaluate the test substance that controls the adverse effects of senescence, such as the suppressive action of the senescence-associated secretory phenotype (SASP). Furthermore, it is also possible to search for a test substance exhibiting an action of rejuvenating senescent cells.

### <Manufacturing method for co-culture product containing human astrocyte cell population, human-derived nerve cells, and human-derived microglia>

According to the present invention, there is provided a manufacturing method for a co-culture product, including a step of adding the human astrocyte cell population, human-derived nerve cells, and human-derived microglia to a culture container, and a step of co-culturing the human astrocyte cell population, the nerve cells, and the microglia in the culture container.

In the present invention, the co-culture product may be any of a two-dimensional culture product or a three-dimensional culture product. The three-dimensional culture product may have a spheroid shape.

In the present invention, in a case where the human astrocyte cell population according to the embodiment of the present invention, the nerve cells, and the microglia are added to a culture container, the timing of adding each cell may be simultaneous or may be separate.

In the present invention, in a case of producing a two-dimensional co-culture product, preferably, the human astrocyte cell population is cultured in advance in a culture container, and the nerve cells and the microglia can be added to the culture container containing the cultured human astrocyte cell population.

In the present invention, in a case of producing a three-dimensional culture product, preferably, the human astrocyte cell population, the nerve cells, and the microglia can be simultaneously added to the culture container prior to the co-culture of the cells.

The "prior to the co-culture of cells" means that the co-culture is not considered to be started within, for example, 1 to 24 hours after the addition of one or two types of cells.

Specifically, for example, in a case of producing a three-dimensional culture product, a suspension and mixture of the three types of cells described above, which are put in one container, may be added to a culture container. In this case, the step of adding the human astrocyte cell population, human-derived nerve cells, and human-derived microglia to a culture container includes a step of suspending the human astrocyte cell population, human-derived nerve cells, and human-derived microglia in a culture medium, and a step of simultaneously adding the culture medium containing the human astrocyte cell population, human-derived nerve cells, and human-derived microglia obtained as described above to the culture container.

Alternatively, the three types of cells described above may be separately suspended in separate culture media and may be simultaneously added to the same culture container. Alternatively, three types of frozen cells frozen and stored separately may be thawed and simultaneously added to the same culture container.

However, the aspect in which the human astrocyte cell population, the nerve cells, and the microglia are simultaneously added to the culture container is not limited to the above.

In the present invention, three types of cells (astrocytes, nerve cells, and microglia) can be co-cultured at any proportion. In addition, by co-culturing three types of cells (astrocytes, nerve cells, and microglia), it is possible to reflect and mimic the human brain more than the conventional culture system, which is useful in studying the original function of the brain and the mechanism of disease formation. In the present invention, since three types of cells are used, it is possible to evaluate the intercellular interaction in brain function or pathogenesis.

The nerve cells and the microglia used for the co-culture with the human astrocyte cell population are preferably obtained by induction of differentiation from human-derived pluripotent stem cells.

Examples of the human-derived pluripotent stem cell include human iPS cells, human ES cells, human mesenchymal stem cells, and the like. Human iPS cells are preferable, but the human-derived pluripotent stem cell is not particularly limited thereto. The human iPS cell is an iPS cell produced from a human cell.

Examples of the human-derived pluripotent stem cell include a pluripotent stem cell derived from a specimen having no mutation in the disease-related gene and a pluripotent stem cell derived from a specimen having a mutation in the disease-related gene, and the human-derived pluripotent stem cell is not particularly limited; however, for example, it is preferably the pluripotent stem cell derived from the specimen having no mutation in the disease-related gene. The "no mutation in disease-related gene" means that there is no mutation that causes a nervous system disease in the disease-related gene. That is, in a case where there is a mutation in a gene but the mutation does not cause a disease, it is interpreted that there is no mutation in the disease-related gene.

The ES cell can be established, for example, by culturing an early embryo before implantation, an inner cell mass constituting the early embryo, a single blastomere, or the like (Manipulating the Mouse Embryo A Laboratory Manual, Second Edition, Cold Spring Harbor Laboratory Press, 1994, Thomson, J. A. et al., Science, 282, 1145-1147, 1998). As the early embryo, an early embryo produced by nuclear transplantation of a nucleus of a somatic cell may be used (Wilmut et al., Nature, 385, 810, 1997, Cibelli et al., Science, 280, 1256, 1998, Iriya et al., Protein Nucleic Acid Enzyme, 44, 892, 1999, Baguisi et al., Nature Biotechnology, 17, 456, 1999, Wakayama et al., Nature, 394, 369, 1998, Nature Genetics, 22, 127, 1999, and Proc. Natl. Acad. Sci. USA, 96, 14984, 1999, Rideout III et al., Nature Genetics, 24, 109, 2000, Tachibana et al., Human Embryonic Stem Cells Derived by Somatic Cell Nuclear Transfer, Cell, 2013, in press). As the early embryo, a parthenogenetic embryo may be used (Kim et al., Science, 315, 482-486, 2007, Nakajima et al., Stem Cells, 25, 983-985, 2007, Kim et al., Cell Stem Cell, 1, 346-352, 2007, Revazova et al., Cloning Stem Cells, 9, 432-449, 2007, Revazova et al., Cloning Stem Cells, 10, 11-24, 2008). In addition to the above-described papers, the production of the ES cell is described in Strelchenko N. et al., Reprod Biomed Online. 9, 623-629, 2004, Klimanskaya I. et al., Nature 444, 481-485, 2006, Chung Y. et al., Cell Stem Cell 2, 113-117, 2008, Zhang X. et al., Stem Cells 24, 2669-2676, 2006, Wassarman, P. M. et al., Methods in Enzymology, Vol. 365, 2003, or the like. It is noted that a fused ES cell obtained by cell fusion of an ES cell with a somatic cell is also included in the embryonic stem cell that is used in the method according to the embodiment of the present invention.

Some ES cells are available from conservation institutions or are commercially available. For example, human ES cells are available from the Institute for Frontier Medical Sciences, Kyoto University (for example, KhES-1, KhES-2, and KhES-3), WiCell Research Institute, ESI BIO.

Examples of the method for obtaining a human-derived nerve cell include inducing from human iPS cells produced from somatic cells collected from healthy humans (healthy person) who do not have a mutation of a disease related gene causing a nervous system disease or who do not have a nervous system disease, or from somatic cells collected from patients who have a mutation of a disease related gene causing a nervous system disease or who have a nervous system disease, and inducing from an established human iPS cell.

The nerve cell differentiated from the human-derived pluripotent stem cell is not particularly limited, but is preferably a motor nerve cell, a cerebral cortex excitatory nerve cell, or a substantia nigra nerve cell.

A method for inducing the differentiation of a nerve cell from a human-derived pluripotent stem cell is not particularly limited, but includes a method of differentiationinduction into a nerve cell after producing a neural stem cell from a pluripotent stem cell using a low-molecular-weight compound treatment or the like, and a method of directly inducing into a nerve cell by gene expression or the like.

Examples of the method for inducing differentiation of a nerve cell from a pluripotent stem cell include
(1) a method of culturing in a serum-free medium to form an embryoid body (a cell mass containing neuronal progenitor cells) and then differentiating (SFEB method: Watanabe K, et al., Nat. Neurosci., 8, 288-296, 2005, SFEBq method: Wataya T, et al., Proc. Natl. Acad. Sci. USA, 105, 11796-11801, 2008),
(2) a method of culturing on stromal cells and differentiating (SDIA method: Kawasaki H, et al., Neuron, 28, 31-40, 2000),
(3) a method of culturing on a Matrigel to which a drug has been added and differentiating (Chambers S.M, et al., Nat. Biotechnol., 27, 275-280, 2009),
(4) a method of culturing in a culture medium containing a low-molecular-weight compound as a substitute for a cytokine and performing differentiation (US5843780A),
(5) a method of differentiating by introducing and expressing a neural-inducing factor (Ngn2, Neurogenin 2, or the like) in pluripotent stem cells (WO2014/148646A, and Zhang Y, et al., Neuron, 78, 785-98, 2013),
(6) a method of differentiating by introducing and expressing miR-9/9*-124 in pluripotent stem cells, and
a combination of these methods.

Among the above, (5) the method of introducing and expressing Ngn2 in pluripotent stem cells is preferable since mature nerve cells can be obtained in a short period of time with high efficiency.

Examples of the human-derived nerve cell include an induced nerve cell differentiated from an iPS cell by forced expression of Ngn2, iCell GlutaNeurons (FUJIFILM Cellular Dynamics, C1033), iCell GABANeurons (FUJIFILM Cellular Dynamics, C1008), iCell DopaNeurons (FUJIFILM Cellular Dynamics, C1028), iCell Motor Neurons (FUJIFILM Cellular Dynamics, C1048), and the like, and the induced nerve cell differentiated from an iPS cell by forced expression of Ngn2 and iCell GlutaNeurons are preferable.

Examples of the human-derived microglia include iCell Microglia (FUJIFILM Cellular Dynamics, C1110), Microglia (Axol Bioscience, AX0664), and the like, and iCell Microglia is preferable.

The nerve cell is preferably a cell which expresses at least one or more marker genes specific to nerve cells consisting of β-III tubulin, NeuN, a neural cell adhesion molecule (N-CAM), and microtubule-associated protein 2 (MAP2), and has a β-III tubulin-positive protrusion (hereinafter, referred to as a neurite).

Microglia are cells that express at least one or more marker genes specific to microglia consisting of ionized calcium-binding adapter molecule 1 (IBA1), CD33, CD45, triggering receptor expressed on myeloid cells 2 (TREM2), purinergic receptor P2Y (P2RY12, G-protein coupled 12), transmembrane protein 119 (TMEM119), and CX3C-chemokine receptor 1 (CX3CR1).

In the present invention, the above-described cells are co-cultured in a culture container.

As the culture container, a plate having wells, a dish, a cell culture insert, a cell culture flask, or the like can be used, and the plate having wells is preferable. Specific examples thereof include ViewPlate (PerkinElmer), a 96-well microplate (Greiner), a 96-well polystyrene microplate (Corning), a PrimeSurface (trademark) plate (Sumitomo Bakelite), a cell-repellent plate (Greiner Bio one), and the like, and ViewPlate (PerkinElmer) and a PrimeSurface (trademark) plate (Sumitomo Bakelite) are preferable.

Examples of the shape of the culture container include a flat bottom, a round bottom, a U-shaped bottom, a V-shaped bottom, and the like, but the shape is not particularly limited.

In a case where the co-culture product is a three-dimensional culture product, the properties of the surface with which the culture medium in the container comes into contact are preferably cell non-adhesiveness. As a result, the cells are cultured in a floating state, and a spheroid is easily formed. Alternatively, only a certain portion of the surface inside the container may be cell adhesiveness, and the other portions may be cell non-adhesiveness. In this case, cells can be gathered at the portion with cell adhesiveness to form a spheroid.

In a case where the co-culture product is a two-dimensional culture product, the lower limit value of the cell density (the cell density is the total cell density of two or more cells to be used, the same applies to the following) at the time of seeding into the culture container is not particularly limited, but it is, for example, preferably 2.5 × 10⁴ cells/cm² or more, more preferably 5.0 × 10⁴ cells/cm² or more, still more preferably 10.0 × 10⁴ cells/cm² or more, even still more preferably 15.0 × 10⁴ cells/cm² or more, particularly preferably 20.0 × 10⁴ cells/cm² or more, and most preferably 25.0 × 10⁴ cells/cm² or more. The upper limit value of the cell density at the time of seeding in the culture container is not particularly limited, but for example, may be 60.0 × 10⁴ cells/cm² or less, and is preferably less than 55.0 × 10⁴ cells/cm², more preferably 50.0 × 10⁴ cells/cm² or less, still more preferably 45.0 × 10⁴ cells/cm² or less, even still more preferably 40.0 × 10⁴ cells/cm² or less, and particularly preferably 35.0 × 10⁴ cells/cm² or less.

In a case where the co-culture product is a two-dimensional culture product, the cell density at the time of seeding in the culture container is preferably 5.0 × 10⁴ cells/cm² or more and 55.0 × 10⁴ cells/cm² or less, more preferably 10.0 × 10⁴ cells/cm² or more and 50.0 × 10⁴ cells/cm² or less, still more preferably 15.0 × 10⁴ cells/cm² or more and 45.0 × 10⁴ cells/cm² or less, even still more preferably 20.0 × 10⁴ cells/cm² or more and 40.0 × 10⁴ cells/cm² or less, and particularly preferably 25.0 × 10⁴ cells/cm² or more and 35.0 × 10⁴ cells/cm² or less.

In a case where the co-culture product is a three-dimensional culture product, the lower limit value of the number of cells (the number of cells is the total number of cells of three types of cells, the same applies to the following) at the time of seeding into the 96-well plate is not particularly limited, but it is, for example, preferably 0.7 × 10⁴ cells/well or more, more preferably 1.0 × 10⁴ cells/well or more, still more preferably 1.2 × 10⁴ cells/well or more, even still more preferably 1.4 × 10⁴ cells/well or more, particularly preferably 1.6 × 10⁴ cells/well or more, and most preferably 1.8 × 10⁴ cells/well or more. The upper limit value of the cell density at the time of seeding in the culture container is not particularly limited, but for example, may be 8.0 × 10⁴ cells/well or less, and is preferably less than 8.0 × 10⁴ cells/well, more preferably 5.0 × 10⁴ cells/well or less, still more preferably 3.0 × 10⁴ cells/well or less, even still more preferably 2.5 × 10⁴ cells/well or less, and particularly preferably 2.3 × 10⁴ cells/well or less.

In a case where the co-culture product is a three-dimensional culture product, the number of cells at the time of seeding into the 96-well plate is preferably 1.0 × 10⁴ cells/well or more and 8.0 × 10⁴ cells/well or less, more preferably 1.2 × 10⁴ cells/well or more and 5.0 × 10⁴ cells/well or less, still more preferably 1.4 × 10⁴ cells/well or more and 3.0 × 10⁴ cells/well or less, even still more preferably 1.6 × 10⁴ cells/well or more and 2.5 × 10⁴ cells/well or less, and particularly preferably 1.8 × 10⁴ cells/well or more and 2.3 × 10⁴ cells/well or less.

A medium can be selected from the known media and commercially available media. The culture medium used for the culture can be used by adding an additive to the basal medium. Here, examples of the basal medium include DMEM, DMEM (Dulbecco's modified Eagle's medium)/F12, BrainPhys Neuronal Medium, Neurobasal Medium-A, Neurobasal Medium, Neural Progenitor Basal Medium, NS-A Basal Medium, Basal Medium Eagle (BME), BGJb Medium, CMRL 1066 Medium, Glasgow Minimum Essential Medium (MEM), Improved MEM Zinc Option, Iscove's Modified Dulbecco's Medium (IMDM), Medium 199, Eagle MEM, αMEM, Ham's F12 Medium, RPMI 1640 Medium, Fischer's Medium, and the like. In addition, as the culture medium, a single culture medium may be used, or two or more culture media may be used in combination.

Specific examples of the additive which can be added to the culture medium include serum, retinoic acid, Wnt, BMP (BMP-4 and the like), CNTF, a glial cell line-derived neurotrophic factor (GDNF), a basic fibroblast growth factor (bFGF), an epidermal growth factor (EGF), a hepatocyte growth factor (HGF), a sonic hedgehog (SHH), insulin-like growth factor 1 (IGF-1), Activin A, Heregulin β-1, interleukins, 8-Br-cAMP, heparin, heparan sulfate, laminin, collagen, fibronectin, progesterone, selenite, B-27 (tradename) supplement, N2 Supplement with Transferrin (Apo), N2 Supplement with Transferrin, GlutaMAX, L(+)-ascorbic acid, ITS-supplement, MEM Non-Essential Amino Acid, and the like, but the additive is not limited thereto. Furthermore, an antibiotic (penicillin, streptomycin, or the like) may be added.

According to the present invention, a co-culture product containing the human astrocyte cell population, the human-derived nerve cells, and the human-derived microglia, which are obtained by the manufacturing method for a co-culture product according to the embodiment of the present invention described above, is provided.

The present invention will be more specifically described with reference to Examples, but the present invention is not limited to the scope of Examples.

### Examples

### Test Example 1: Induction from human iPS cell-derived astrocyte progenitor cells to human senescent astrocytes

### (1) Senescence induction by long-term culture of astrocyte progenitor cells

Human iPS cell-derived astrocyte progenitor cells were purchased from Axol Bioscience (ax0083), Applied Stem Cell (ASE-9322P), or XCell Science (XCS-AP-001-1V) and used.

15 mL of iMatrix-511 silk (Matrixome, 892021) diluted 167 times with PBS (-) (FUJIFILM Wako Pure Chemical Corporation, 166-23555) was added to a flask (CELLCOAT (registered trademark), PDL, 650 ml, flask, filter cap, Greiner Bio One, 661940), and the flask was allowed to stand at 4°C. After 24 hours, the culture medium was replaced with the following progenitor cell culture medium and used.

### Composition of progenitor cell culture medium

The reagents shown in Table 2 were added to DMEM/F12 (Life technologies, 11320-033).

**[Table 2]**

| Reagent name | Manufacturer and model number | Final concentration |
|---|---|---|
| N2 Supplement with Transferrin (Apo) (×100) | FUJIFILM Wako Pure Chemical Corporation, 141-09041 | ×1 |
| GlutaMAX | Thermo Fisher Scientific, 35050-061 | 1% |
| MEM Non-Essential Amino Acid | Thermo Fisher Scientific, 11140-050 | 1% |
| Penicillin-Streptomycin Solution (×100) | FUJIFILM Wako Pure Chemical Corporation, 168-23191 | ×0.5 |
| Heparin Sodium | FUJIFILM Wako Pure Chemical Corporation, 085-00134 | 0.5U/mL |
| Animal-Free Recombinant Human FGF-basic | PeproTech, AF-100-18B | 10ng/mL |
| Animal-Free Recombinant Human EGF | PeproTech, AF-100-15 | 10ng/mL |

Human iPS cell-derived astrocyte progenitor cells were suspended in a progenitor cell culture medium, seeded in a flask at a density of 30 × 10⁴ cells/flask, and cultured under the conditions of 37°C and 5% CO₂. Subculturing was performed every two weeks, and the culture was continued for 84 days.

### (2) Differentiation induction into human senescent astrocytes

Matrigel basement membrane matrix (Corning, 356234) diluted 120-fold with DMEM/F12 (Life technologies, 11320-033) was added to a 6-well plate at 1.5 mL/well and allowed to stand at 4°C. After 24 hours, the culture medium was replaced with a progenitor cell culture medium and used.

Human iPS cell-derived astrocyte progenitor cells cultured for 84 days were peeled with TrypLE Select (Thermo Fisher Scientific, 12563-029), seeded into a 6-well plate at a density of 30 × 10⁴ cells/well, and cultured under the conditions of 37°C and 5% CO₂. The next day, the culture medium was replaced with the following differentiation-inducing culture medium and further cultured for 5 days to induce differentiation into human senescent astrocytes.

### Composition of differentiation-inducing culture medium

The reagents shown in Table 3 were added to DMEM/F12 (Life technologies, 11320-033).

**[Table 3]**

| Reagent name | Manufacturer and model number | Final concentration |
|---|---|---|
| N2 Supplement with Transferrin (Apo) (×100) | FUJIFILM Wako Pure Chemical Corporation, 141-09041 | ×1 |
| GlutaMAX | Thermo Fisher Scientific, 35050-061 | 1% |
| MEM Non-Essential Amino Acid | Thermo Fisher Scientific, 11140-050 | 1% |
| Penicillin-Streptomycin Solution (×100) | FUJIFILM Wako Pure Chemical Corporation, 168-23191 | ×0.5 |
| Heparin Sodium | FUJIFILM Wako Pure Chemical Corporation, 085-00134 | 0.5U/mL |
| Animal-Free Recombinant Human CNTF | PeproTech, AF-450-13 | 10ng/mL |
| Animal-Free Recombinant Human BMP-4 | PeproTech, AF-120-05ET | 10ng/mL |

### (3) Expression of astrocyte marker

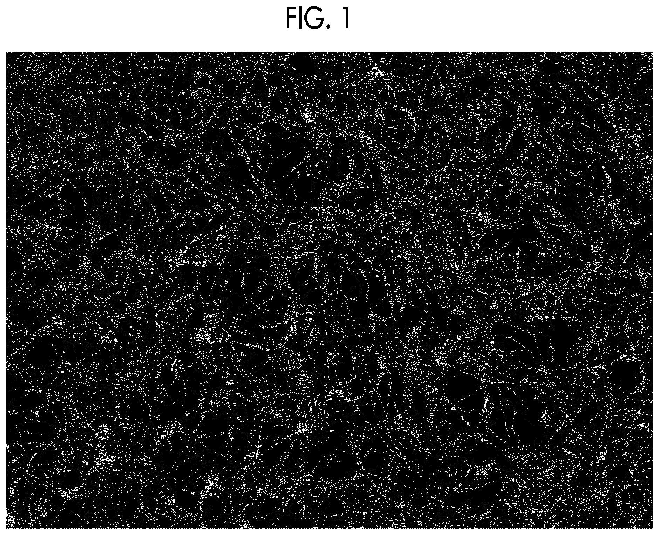

The expression of GFAP, which is a marker of astrocytes, was confirmed by immunostaining. Human astrocytes produced from senescent human astrocyte progenitor cells and human astrocytes produced from non-senescent human astrocyte progenitor cells were fixed by adding a formaldehyde solution (FUJIFILM Wako Pure Chemical Corporation, 061-00416) diluted 10 times with PBS (-) (FUJIFILM Wako Pure Chemical Corporation, 166-23555) and allowing to stand at room temperature for 30 minutes. After washing three times with PBS (-), blocking was performed by adding 1% bovine serum albumin (BSA) (1% BSA) to PBS (-). The blocking liquid was removed, and a primary antibody (Millipore, MAB3402) diluted 3,000-fold with 1% BSA was added thereto, and the mixture was allowed to stand overnight at 4°C. After washing 3 times with PBS (-), a secondary antibody (ThermoFisher Scientific, A11005) diluted 1,000 folds with 1% BSA was added thereto, and the mixture was treated at room temperature for 1 hour. After washing 3 times with PBS (-), images were acquired by photographing with IncuCyte (registered trademark) S3 (Essen BioScience, 4647). The results are shown in Fig. 1.

### (4) Quantification of the number of human astrocytes by flow cytometer

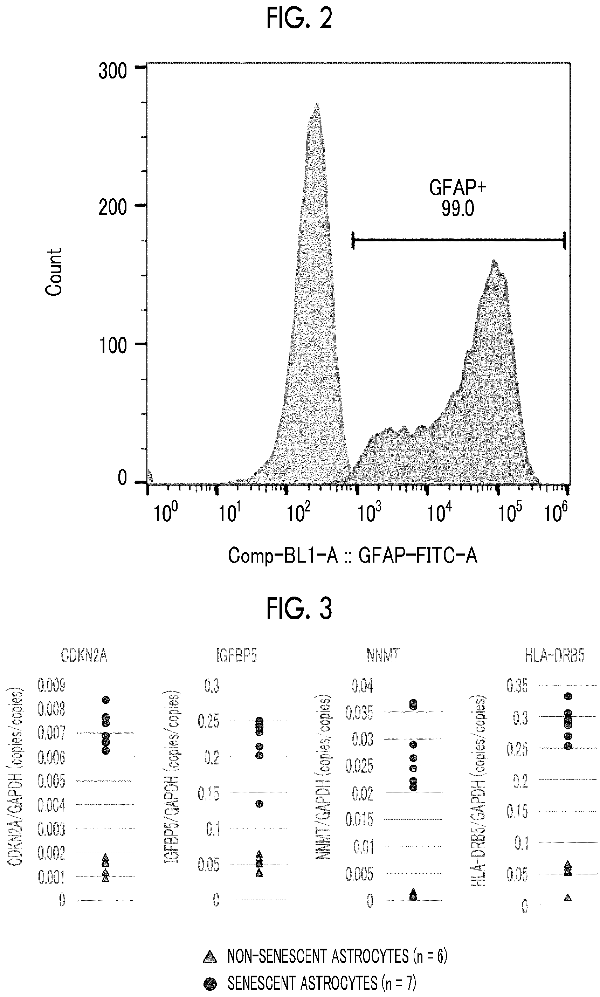

For human astrocytes produced from senescent human astrocyte progenitor cells, the GFAP positive rate was quantified by a flow cytometer. The living cells and the dead cells were labeled using a LIVE/DEAD Fixable Aqua Dead Cell Stain Kit (Thermo Fisher Scientific, L34957) according to the attached document. A 2% formaldehyde solution (FUJIFILM Wako Pure Chemical Corporation, 061-00416) was added to the cells, and the cells were allowed to stand for 15 minutes and fixed. After washing with Perm/Wash Buffer (BD Bioscience, 554723), the cells were labeled with GFAP Monoclonal Antibody (131-17719), Alexa Fluor (registered trademark) 488 (Thermo Fisher Scientific, A-21294), or Alexa Fluor (registered trademark) 488 Mouse IgG1, κ Isotype Ctrl (FC) Antibody (Biolegend, 400129), and then only living cells were selected using a Attune NxT Flow Cytometer (Thermo Fisher Scientific) with a signal of LIVE/DEAD Fixable Aqua Dead Cell Stain as an indicator, and the fluorescence signal intensity in a case of being irradiated with a 488 nm laser and the number (count) of cells having the signal intensity were obtained (Fig. 2). For cells treated with GFAP Monoclonal Antibody, Alexa Fluor 488, cells that exhibited fluorescence stronger than the fluorescence signal intensity in a case of being treated with a negative control Alexa Fluor 488 Mouse IgG1, κ Isotype Ctrl (FC) Antibody that does not respond to a specific endogenous antigen were defined as GFAP-positive, and the proportion of GFAP-positive cells to the total number of living cells was calculated as the GFAP-positive rate.

99% of human astrocytes produced from senescent human astrocyte progenitor cells were GFAP-positive.

### (5) Quantification of marker expression by digital PCR

Total RNA was extracted from human senescent astrocytes. For the extraction of total RNA, RNeasy (registered trademark) Plus Mini Kit (Qiagen, 74136) was used according to the attached document. For the synthesis of cDNA from total RNA, a PrimeScript RT reagent Kit with gDNA Eraser (Perfect Real Time) (Takara Bio, RR047A) was used according to the attached document.

For the quantification of the marker, ddPCR EvaGreen Supermix (Bio-Rad, 1864034), which is droplet digital PCR, was used. Samples were prepared with the composition shown in Table 4, and the marker expression level was absolutely quantified using a QX200 AutoDG Droplet Digital PCR system (Bio-Rad, 1864100J3).

**[Table 4]**

| Reagent name | Final concentration |
|---|---|
| 2× QX200 ddPCR | 1× |
| Evagreen Supermix | |
| Forward primer | 136 nM |
| Reverse primer | 136 nM |
| DNA template | 9.1 ng (For CDKN2A) |
| | 0.45 ng (For GAPDH) |

The primers described in Table 5 below were used.

**[Table 5]**

| Gene name | Sequence | Manufacturer |
|---|---|---|
| GAPDH | Fw: undisclosed | Takara Bio, HA067812 |
| | Rv: undisclosed | |
| CDKN2A | Fw: CACATTCATGTGGGCATTTC (SEQ ID NO: 1) | Takara Bio |
| | Rv: AGCTTTGGTTCTGCCATTTGCTA (SEQ ID NO: 2) | |
| IGFBP5 | Fw: TTGCCTCAACGAAAAGAGCT (SEQ ID NO: 3) | Fasmac |
| | Rv: CGGTCCTTCTTCACTGCTTC (SEQ ID NO: 4) | |
| NNMT | Fw: TGGCCCCACTATCTATCAGC (SEQ ID NO: 5) | Fasmac |
| | Rv: TGGACCCTTGACTCTGTTCC (SEQ ID NO: 6) | |
| HLA-DRB5 | Fw: GTGAGACTTGCCTGCTCCTC (SEQ ID NO: 7) | Fasmac |
| | Rv: CGTCCCGTTGAAGAAATGAC (SEQ ID NO: 8) | |
| C3 | Fw: GTGGAAATCCGAGCCGTTCTCT (SEQ ID NO: 9) | Fasmac |
| | Rv: GATGGTTACGGTCTGCTGGTGA (SEQ ID NO: 10) | |

A PCR reaction was performed under the conditions of Table 6 below, and the expression level was calculated as an expression level (copies/copies) per expression level of GAPDH.

**[Table 6]**

| Step | Temperature (°C) | Time | Ramp rate | Number of Cycles |
|---|---|---|---|---|
| Enzyme activation | 95 | 5 min | 2 °C/sec | 1 |
| Denaturation | 95 | 30 sec | | 40 |
| Annealing/extension | 60 | 1 min | | 40 |
| Signal stabilization | 4 | 5 min | | 1 |
| | 90 | 5 min | | 1 |
| Hold | 4 | infinite | | 1 |

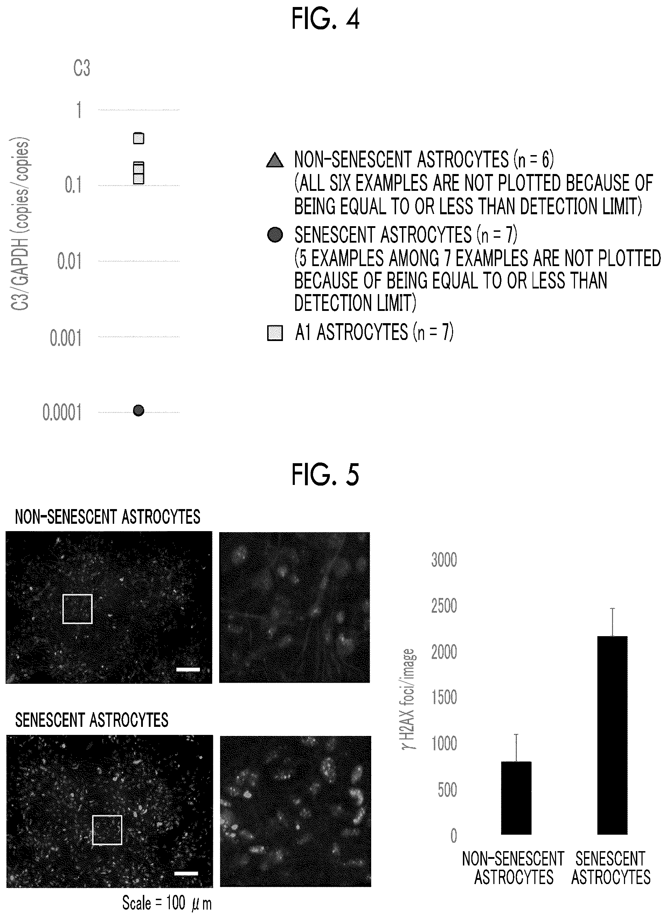

The results are shown in Figs. 3 and 4. It was confirmed that CDKN2A, IGFBP5, NNMT, and HLA-DRB5 were highly expressed in the senescent human astrocytes as compared with the non-senescent human astrocytes. In addition, it was confirmed that the senescent human astrocytes and the non-senescent human astrocytes hardly expressed C3 as compared with the human A1 astrocytes. C3 could not be detected in all of the six cases of the non-senescent human astrocytes. C3 could not be detected in 5 out of 7 cases of senescent human astrocytes. That is, it was confirmed that the senescent human astrocytes and the non-senescent human astrocytes are not the human A1 astrocytes.

### (6) Staining of senescent marker (γH2AX)

The focus formation of γH2AX, which is one of the senescent markers, was evaluated by immunostaining. Human astrocytes produced from senescent human astrocyte progenitor cells and human astrocytes produced from non-senescent human astrocyte progenitor cells were fixed by adding a formaldehyde solution (FUJIFILM Wako Pure Chemical Corporation, 061-00416) diluted 10 times with PBS (-) (FUJIFILM Wako Pure Chemical Corporation, 166-23555) and allowing to stand at room temperature for 30 minutes. After washing 3 times with PBS (-), the astrocytes were blocked by adding a PBS (-) solution (1% BSA) containing 1% BSA, and a primary antibody (Millipore, O5-636-I) diluted 3,000-fold with 1% BSA was added thereto, and the mixture was allowed to stand overnight at 4°C. After washing 3 times with PBS (-), a secondary antibody (ThermoFisher Scientific, A11029) diluted 1,000 folds with 1% BSA was added thereto, and the mixture was treated at room temperature for 1 hour. After washing 3 times with PBS (-), images were acquired by photographing with IncuCyte (registered trademark) S3 (Essen BioScience, 4647). The number of foci (dot-like signals) was quantified using ImageJ. The fluorescence signal was binarized (threshold value: 70), and the number of foci (dots) was quantified by "Analyze Particles". The results are shown in Fig. 5.

It was confirmed that in the senescent human astrocyte, more foci of γH2AX, which is one of the senescent markers, were formed as compared with the non-senescent human astrocytes.

### (7) Staining of senescent marker (SA-β-GAL)

The expression of SA-β-GAL, which is one of the senescent markers, was evaluated. Human astrocytes produced from senescent human astrocyte progenitor cells and human astrocytes produced from non-senescent human astrocyte progenitor cells were fixed by adding a formaldehyde solution (FUJIFILM Wako Pure Chemical Corporation, 061-00416) diluted 10 times with PBS (-) (FUJIFILM Wako Pure Chemical Corporation, 166-23555) and allowing to stand at room temperature for 30 minutes. For the expression of SA-β-GAL, Cellular Senescence Detection Kit-SPiDER-βGal (Dojindo Laboratories, Inc., SG03) was used according to the attached document. The images were acquired by imaging with IncuCyte (registered trademark) S3 (Essen BioScience, 4647), and the positive area (total area) and the fluorescence intensity (total integrated intensity) were quantified using Basic Analysis software. The result is shown in Fig. 6.

It was confirmed that SA-β-GAL was strongly expressed in senescent human astrocytes as compared with non-senescent human astrocytes.

### Test Example 2: Comparison of senescent markers in long-term culture of human astrocyte progenitor cell and human astrocyte

Human iPS cell-derived astrocyte progenitor cells purchased from Axol Bioscience (ax0083), Applied Stem Cell (ASE-9322P), or XCell Science (XCS-AP-001-1V) were used.

Human iPS cell-derived astrocyte progenitor cells were cultured in a progenitor cell culture medium for 43 days, and then the culture medium was replaced with a differentiation-inducing culture medium to induce differentiation into human astrocytes ((1) in Fig. 7). Furthermore, the total RNA was extracted from cells obtained by culturing the cells obtained in (1) for 42 days ((2) in Fig. 7) and cells obtained by culturing human iPS cell-derived astrocyte progenitor cells in a progenitor cell culture medium for 85 days and then replacing the culture medium with a differentiation-inducing culture medium to induce differentiation into human astrocytes ((3) in Fig. 7) using RNeasy (registered trademark) Plus Mini Kit (Qiagen, 74136) according to the attached document. The expression of CDKN2A, which was a senescent marker, was absolutely quantified according to the same method as in (5) Quantification of marker expression by digital PCR of Test Example 1. The result is shown in Fig. 7.

The expression level of CDKN2A in human astrocytes produced from human iPS cell-derived astrocyte progenitor cells cultured for 85 days was 0.0109 copies/copies, whereas the expression level of CDKN2A in human astrocytes produced from human iPS cell-derived astrocyte progenitor cells cultured for 43 days and further cultured for 42 days was 0.0025 copies/copies. From this, it was found that it is important to perform the culture in a state of astrocyte progenitor cells for a long period of time to produce senescent human astrocytes.

### Test Example 3: Production of two-dimensional co-culture product of senescent astrocytes, nerve cells, and microglia

### (1) Senescence induction by long-term culture of astrocyte progenitor cells and differentiation induction into human senescent astrocytes

Human iPS cell-derived astrocyte progenitor cells were purchased from Axol Bioscience (ax0083), Applied Stem Cell (ASE-9322P), or XCell Science (XCS-AP-001-1V) and used.

Matrigel basement membrane matrix (Corning, 356234) diluted 120-fold with DMEM/F12 (Life technologies, 11320-033) was added to a 96-well plate at 65 µL/well and allowed to stand at 4°C. After 24 hours, the culture medium was replaced with a progenitor cell culture medium and used. The progenitor cell culture medium was prepared with the same composition as that of Table 2 of Test Example 1.

Human iPS cell-derived astrocyte progenitor cells cultured for 84 days were seeded in a 96-well plate at a density of 5 × 10⁴ cells/well and cultured under the conditions of 37°C and 5% CO₂. The next day, the culture medium was replaced with the differentiation-inducing culture medium and further cultured for 5 days to induce differentiation into senescent astrocytes. The differentiation-inducing culture medium was prepared with the same composition as that of Table 3 of Test Example 1.

### (2) Production of two-dimensional co-culture product

The nerve cells were produced by forcibly expressing the Ngn2 gene from iPS cells (Chao Wang, et al., Stem Cell Reports, 9: 1221-1233, 2017), and nerve cells that had been frozen and stored were used. The cells that had been frozen and stored were thawed in a warm bath at 37°C, and after thawing, the cells were added to the co-culture medium and centrifuged at 600 × g at room temperature for 5 minutes. After centrifugation, the supernatant was removed, the cells were suspended in 1 mL of the co-culture medium, and the number of cells was counted.

### Composition of co-culture medium

The reagents shown in Table 7 were added to DMEM/F12 (Life technologies, 11320-033).

**[Table 7]**

| Reagent name | Manufacturer and model number | Final concentration |
|---|---|---|
| N2 Supplement with Transferrin (Apo) (×100) | FUJIFILM Wako Pure Chemical Corporation, 141-09041 | ×1 |
| GlutaMAX | Thermo Fisher Scientific, 35050-061 | 1% |
| MEM Non-Essential Amino Acid | Thermo Fisher Scientific, 11140-050 | 1% |
| Penicillin-Streptomycin Solution (×100) | FUJIFILM Wako Pure Chemical Corporation, 168-23191 | ×0.5 |
| Animal-Free Recombinant Human CNTF | PeproTech, AF-450-13 | 10 ng/mL |
| Animal-Free Recombinant Human BMP-4 | PeproTech, AF-120-05ET | 10 ng/mL |

As the microglia, iPS cell-derived microglia (iCell Microglia, FCDI, C1110) was used. The cells that had been frozen and stored were thawed in a warm bath at 37°C, and after thawing, the cells were added to the co-culture medium and centrifuged at 600 × g at room temperature for 5 minutes. After centrifugation, the supernatant was removed, the cells were suspended in 1 mL of the co-culture medium, and the number of cells was counted.

The nerve cells and the microglia were seeded on the senescent astrocytes such that the number of each was 3 × 10⁴ cells/well. The cells were cultured under the conditions of 37°C and 5% CO₂, and half of the culture medium was replaced three times a week.

### (3) Cell fixation

The two-dimensional co-culture product that had been cultured for 3 weeks was fixed by treating with a formaldehyde solution (FUJIFILM Wako Pure Chemical Corporation, 061-00416) diluted 10 times with PBS (-) (FUJIFILM Wako Pure Chemical Corporation, 166-23555) for 30 minutes.

### (4) Immunostaining

The fixed cells were washed with PBS (-), and then subjected to blocking and permeation treatment by treating the cells with a solution (1% BSA/0.2% Triton X-100) obtained by diluting BSA (Sigma Aldrich, A4161) and Triton X-100 (BioVision, 2104-100) with PBS (-) to 1% and 0.2% for 30 minutes. Thereafter, as a primary antibody reaction, an anti-MAP2 antibody (Novus Biologicals, NB300-213) diluted 800 times, an anti-GFAP antibody (Millipore, MAB3402) diluted 3,000 times, and an anti-IBA antibody (FUJIFILM Wako Pure Chemical Corporation, 011-27991) diluted 800 times with 1% BSA/0.2% Triton X-100 were treated and allowed to stand overnight at 4°C.

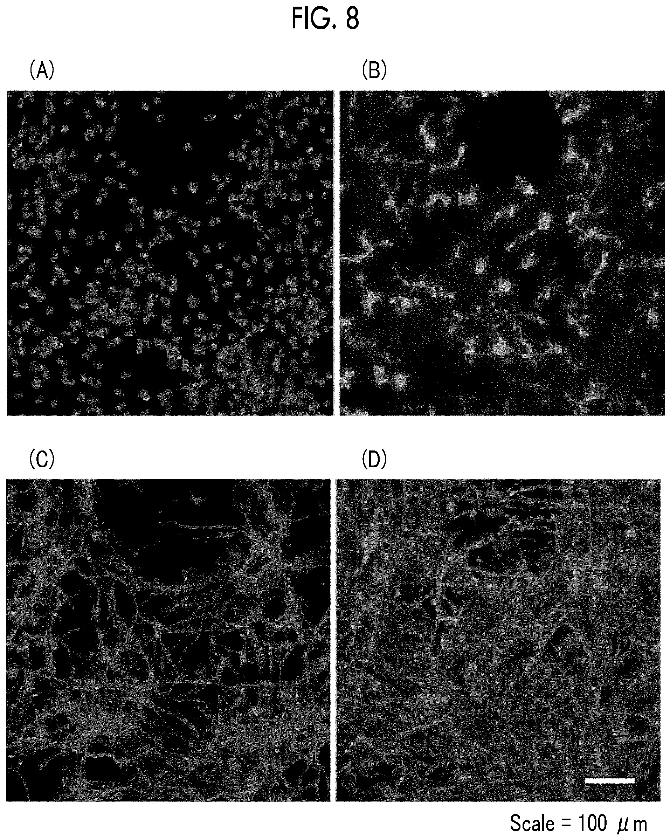

The next day, after washing with PBS (-), as a secondary antibody reaction, Goat antirabbit Alexa Fluor 488 (Thermo Fisher Scientific, Inc., A11008) diluted 1,000 times, Goat antichicken Alexa Fluor 594 (Thermo Fisher Scientific, Inc., A11042) diluted 1,000 times, Goat anti-mouse Alexa Fluor 647 (Thermo Fisher Scientific, Inc., A32728) diluted 1,000 times, and Hoechst 33342 (Dojindo, 346-07951) diluted 1,000 times were treated and allowed to stand at room temperature for 60 minutes. After washing with PBS (-), the cells were imaged with a fluorescence microscope (Nikon, ECLIPSE Ti) to acquire an image. Fig. 8 shows the acquired image. (A) indicates nuclei (Hoechst), (B) indicates microglia (IBA1), (C) indicates nerve cells (MAP2), and (D) indicates astrocytes, all of which were stained, and it was possible to detect signals in any of the cells.

### Test Example 4: Production of three-dimensional co-culture product of senescent astrocytes, nerve cells, and microglia

### (1) Senescence induction by long-term culture of astrocyte progenitor cells and differentiation induction into human senescent astrocytes

Human iPS cell-derived astrocyte progenitor cells (XCell Science, XCS-AP-001-1V) cultured for 84 days were seeded in a flask coated with Matrigel basement membrane matrix (Corning, 354234) and cultured for 5 days (37°C, 5% CO₂) using a differentiation-inducing culture medium to induce differentiation into astrocytes. The differentiation-inducing culture medium was prepared with the same composition as that of Table 3 of Test Example 1.

### (2) Production of three-dimensional co-culture product

The differentiated astrocytes were peeled by TrypLE Select (Thermo Fisher Scientific, 12563-029) and centrifuged at 600 × g at room temperature for 5 minutes. After centrifugation, the supernatant was removed, the cells were suspended in 1 mL of the 3D co-culture medium, and the number of cells was counted.

The nerve cells produced by forcibly expressing the Ngn2 genes from the iPS cells in the same manner as in Test Example 3 was thawed in a warm bath at 37°C. After thawing, the cells were added to the 3D co-culture medium and centrifuged at 600 × g at room temperature for 5 minutes. After centrifugation, the supernatant was removed, the cells were suspended in 1 mL of the 3D co-culture medium, and the number of cells was counted.

The iPS cell-derived microglia (iCell Microglia, FCDI, C1110) were thawed in a warm bath at 37°C. After thawing, the cells were added to the 3D co-culture medium and centrifuged at 600 × g at room temperature for 5 minutes. After centrifugation, the supernatant was removed, the cells were suspended in 1 mL of the 3D co-culture medium, and the number of cells was counted.

The astrocytes, the nerve cells, and the microglia were mixed at a ratio of 10:6:3, and seeded into a 96-well plate (PrimeSurface (registered trademark) plate 96U, Sumitomo Bakelite, MS-9096U) at a density of 1.9 × 10⁴ cells/well. The cells were cultured under the conditions of 37°C and 5% CO₂, and half of the culture medium was replaced three times a week.

### Composition of 3D co-culture medium

The reagents shown in Table 8 were added to DMEM/F12 (Life technologies, 11320-033).

**[Table 8]**

| Reagent name | Manufacturer and model number | Final concentration |
|---|---|---|
| N2 Supplement with Transferrin (Apo) (×100) | FUJIFILM Wako Pure Chemical Corporation, 141-09041 | ×1 |
| GlutaMAX | Thermo Fisher Scientific, 35050-061 | 1% |
| MEM Non-Essential Amino Acid | Thermo Fisher Scientific, 11140-050 | 1% |
| Penicillin-Streptomycin Solution (×100) | FUJIFILM Wako Pure Chemical Corporation, 168-23191 | ×0.5 |
| Animal-Free Recombinant Human CNTF | PeproTech, AF-450-13 | 10 ng/mL |
| Animal-Free Recombinant Human BMP-4 | PeproTech, AF-120-05ET | 10 ng/mL |
| 8-Br-cAMP | Sigma Aldrich, B7880 | 1 µM |
| Animal-Free Recombinant Human/Murine/Rat BDNF | Peprotech, AF-450-02 | 20 ng/mL |
| Recombinant Human GDNF | Peprotech, 450-10 | 20 ng/mL |
| L (+)-Ascorbic Acid | FUJIFILM Wako Pure Chemical Corporation, 012-04802 | 20 nM |
| Laminin | Sigma Aldrich, L2020 | 1 µg/mL |
| B-27 Supplement (50X), serum free | Thermo Fisher Scientific 17504044 | ×0.5 |

### (3) Cell fixation and immunostaining

The three-dimensional co-culture product that had been cultured for 3 weeks was fixed by treating with 4% paraformaldehyde-phosphate buffer solution (FUJIFILM Wako Pure Chemical Corporation, 161-20141) for 30 minutes. After washing with PBS (-), the co-culture product was treated with Triton X-100 (BioVision, 2104-100) diluted to 0.4% with PBS (-) for 15 minutes to perform permeation treatment. After the permeation treatment, the co-culture product was treated with a BSA solution (Sigma-Aldrich, A416) prepared at a concentration of 4% with PBS (-) for 30 minutes to perform blocking. Then, as a primary antibody reaction, an anti-MAP2 antibody (Novus, NB300-213) diluted 600-fold, an anti-IBA1 antibody (FUJIFILM Wako Pure Chemical Corporation, 019-19741) diluted 600-fold, and an anti-GFAP antibody (Merck, MAB3402) diluted 3,000-fold were treated and allowed to stand overnight at 4°C.

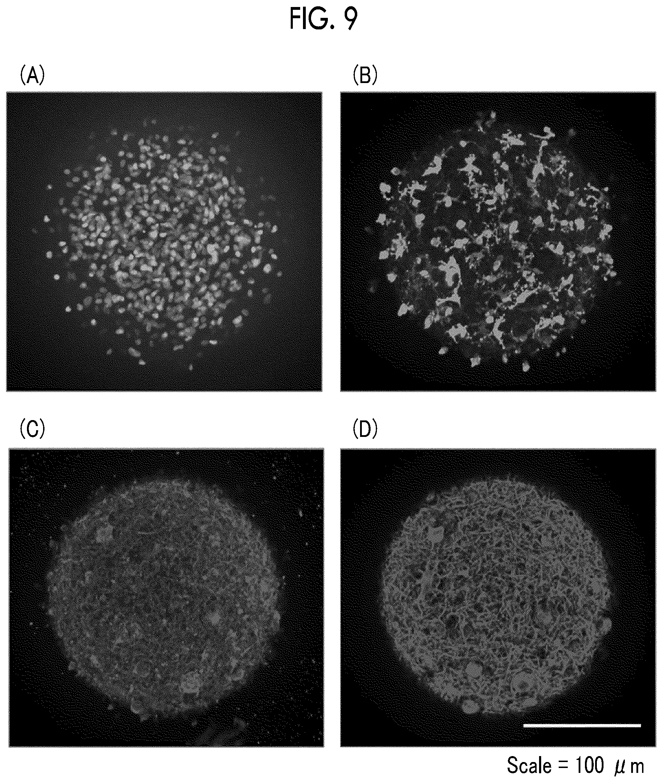

The next day, after washing with PBS (-), as a secondary antibody reaction, Goat antirabbit Alexa Fluor 488 (Thermo Fisher Scientific, Inc., A11008) diluted 1,000 times, Goat anti-Chicken Alexa Fluor 594 (Thermo Fisher Scientific, Inc., A11042) diluted 1,000 times, Goat anti-Mouse Alexa Fluor 647 (Thermo Fisher Scientific, Inc., A32728) diluted 1,000 times, and Hoechst 33342 solution (Dojindo, H342) diluted 1,000 times were treated and allowed to stand at room temperature for 60 minutes. After washing with PBS (-), imaging was performed with a confocal quantitative image cytometer CQ1 (Yokogawa Electric Corporation, Cell Voyager CQ1) to acquire images. Fig. 9 shows the acquired image. (A) indicates nuclei (Hoechst), (B) indicates microglia (IBA1), (C) indicates nerve cells (MAP2), and (D) indicates astrocytes, all of which were stained, and it was possible to detect all signals.

[Sequence list] International application 22F01243W1JP23035597_8.xml based on International Patent Cooperation Treaty

## Claims

1. A human astrocyte cell population that is differentiated from astrocyte progenitor cells derived from human iPS cells, the human astrocyte cell population comprising:
at least 90% of human astrocytes,
wherein in the human astrocytes,
a) CDKN2A is positive,
b) at least one gene marker selected from the group consisting of IGFBP5, NNMT, HLA-DRB 1, and HLA-DRB5 is positive, and
c) an expression level of C3, which is standardized with GAPDH of a reference gene, is 0.05 copies/copies or less.

2. The human astrocyte cell population according to claim 1,
wherein in the human astrocytes, an expression level of CDKN2A, which is standardized with GAPDH of the reference gene, is 0.004 copies/copies or more.

3. The human astrocyte cell population according to claim 1 or 2,
wherein in the human astrocytes, an expression level of IGFBP5, which is standardized with GAPDH of the reference gene, is 0.1 copies/copies or more.

4. The human astrocyte cell population according to claim 1 or 2,
wherein in the human astrocytes, an expression level of NNMT, which is standardized with GAPDH of the reference gene, is 0.005 copies/copies or more.

5. The human astrocyte cell population according to claim 1 or 2,
wherein in the human astrocytes, an expression level of HLA-DRB5, which is standardized with GAPDH of the reference gene, is 0.1 copies/copies or more.

6. The human astrocyte cell population according to claim 1 or 2,
wherein at least one gene marker selected from the group consisting of γH2AX and SA-β-GAL is further positive.

7. The cell population according to claim 1 or 2,
wherein the astrocyte progenitor cells derived from human iPS cells are astrocyte progenitor cells produced from human iPS cells derived from a healthy person.

8. A cell population culture product comprising:
the human astrocyte cell population according to claim 1 or 2; and
a culture medium that does not substantially contain serum.

9. The cell population culture product according to claim 8, further comprising:
at least one factor selected from the group consisting of BMP4 and CNTF.

10. The manufacturing method for the astrocyte cell population according to claim 1 or 2, comprising:
proliferating astrocyte progenitor cells derived from human iPS cells; and
inducing the differentiation of the proliferated astrocyte progenitor cells derived from human iPS cells.

11. An evaluation method for a test substance, comprising:
bringing the human astrocyte cell population according to claim 1 or 2 into contact with a test substance.

12. A manufacturing method for a co-culture product, comprising:
a step of adding the human astrocyte cell population according to claim 1 or 2, human-derived nerve cells, and human-derived microglia to a culture container; and
a step of co-culturing the human astrocyte cell population, the nerve cells, and the microglia in the culture container.

13. The manufacturing method according to claim 12,
wherein the nerve cells and the microglia are obtained by inducing differentiation from human-derived pluripotent stem cells.

14. The manufacturing method according to claim 12 or 13,
wherein human-derived pluripotent stem cells are human iPS cells.

15. The manufacturing method according to claim 12 or 13,
wherein the co-culture product is a two-dimensional culture product or a three-dimensional culture product.

16. A co-culture product which is obtained by the manufacturing method according to claim 12 or 13; comprising:
the human astrocyte cell population according to claim 1 or 2;
human-derived nerve cells; and
human-derived microglia.
